# EUROPEAN PATENT APPLICATION

(11) **EP 0 933 431 A1**
(43) Date of publication of application: **04.08.1999**
(21) Application number: 97930727.9
(22) Date of filing: 09.07.1997
(51) Int. Cl.: C12Q 1/68, C12N 15/11

(54) **METHOD FOR EXAMINING NUCLEIC ACIDS AND EXAMINATION KITS**

(30) Priority: 11.07.1996 JP 20117696
(71) Applicant: WAKUNAGA PHARMACEUTICAL CO., LTD., Osaka-shi, Osaka-fu 532 (JP); SRL, INC., Tachikawa-shi, Tokyo 190 (JP)
(72) Inventor: OKA, Takanori-Wakunaga Pharmaceutical Co., Ltd., Hiroshima 739-11 (JP)
(74) Representative: Kremer, Simon Mark
(86) International application number: JP9702370
(87) International publication number: WO9802574

(57) **Abstract**

Gene mutations or polymorphisms are surely detected, identified or determined by simple procedures within a short period of time directly from specimens containing the gene mutations or polymorphisms in a trace amount within specific regions in target nucleic acids. A method for examining nucleic acids which comprises amplifying a specific region of a target nucleic acid in a specimen to thereby prepare a double stranded sample DNA, adding an excessive amount of the above-mentioned sample DNA to a labeled standard DNA consisting of a double stranded nucleic acid which has a site capable of binding to a solid phase carrier in one strand and a detectable label on another strand to effect competitive hybridization, detecting the above-mentioned labeled standard DNA thus reconstituted with the use of the detectable label and the site capable of binding to a solid phase carrier, and thus determining the degree of the substitution of the complementary strands between the above-mentioned sample DNA and the above-mentioned labeled standard DNA to thereby detect the target DNA which is identical with the above-mentioned labeled standard DNA contained in the above-mentioned sample DNA, characterized in that the detection limit of the target DNA which is identical with the above-mentioned labeled standard DNA contained in the above-mentioned sample DNA is preliminarily specified and, in the step of the competitive hybridization, the extent of the excessiveness of the above-mentioned sample DNA to be added to the above-mentioned labeled standard DNA is specified depending on the detection limit thus specified; and examination kits for detecting, identifying and determining nucleic acids carrying gene mutations or polymorphisms in accordance with this examination method.

## Description

### TECHNICAL FIELD

This invention relates to a nucleic acid assay process adapted for use in detecting, identifying or quantitating the nucleic acid including mutation or polymorphism of the gene, and an assay kit. More specifically, this invention relates to a nucleic acid assay process which is capable of reliably and directly detecting, identifying or quantitating the mutation or the polymorphism of a gene by a simple operation in a short period from a specimen wherein a minute amount of the analyte nucleic acid has a mutation or polymorphism of the gene in the particular region of the analyte nucleic acid, and an assay kit for conducting such assay process.

### BACKGROUND TECHNOLOGY

Today, it has been revealed that substantially all of various enzyme deficiencies which had been known as congenital dysbolism for quite some time are gene diseases. In the diagnosis of such diseases, it is quite important to detect whether the mutation or the polymorphism is present on one of the alleles (hetero) or on both of the alleles (homo).

In the case of diseases which had been induced by acquired gene abnormality, namely, cancer, a particular gene in the cell undergoes mutation and uncontrolled propagation of the cell finally invites death of the individual. When a cancer tissue is used for the specimen, detection of gene mutation or polymorphism at high a sensitivity is required despite the situation that the abnormal cells (cancer cells) of a minute amount is copresent with a large amount of normal cells. When a correlation is present between the type of the mutation or polymorphism and the progress or prognosis of the disease, not only the presence of the gene mutation or polymorphism but the identification of the mutant or polymorphic gene is effective and necessary. If the percentage (content) of such mutant or polymorphic gene in relation to the normal gene within the tissue section could also be quantitated, progress and prognosis of the cancer can be monitored, and such monitoring should greatly facilitate the diagnosis and treatment of the cancer.

The genes which have been pointed out to have some relation to canceration (cancer-related genes) include k-ras gene, N-ras gene, p53 gene, BRCA1 gene, BRCA2 gene, and APC gene, and among such cancer-related genes, many studies have been conducted for those wherein site and type of the mutation or polymorphism are relatively limited, and those wherein frequency of occurrence is deviated to some particular sites in spite of the diverse site and type of the variation.

For example, in the case of the k-ras gene, site of the mutation is concentrated on twelfth codon, and a correlation as high as 80% is known to exist between the pancreatic cancer and the presence of mutation in the k-ras gene. Detection of the mutation in the k-ras gene should enable diagnosis and treatment of the pancreatic cancer at an early stage.

In the case of the p53 gene, the type and the site of the mutation or the polymorphism are not limited to such extent. The variation at 175th, 248th and 273rd codons, however, comprises about 30% of the total variation, and clarification of the correlation between the type and the site of the mutation or the polymorphism and the particular types of cancers is expected to enable diagnosis and treatment of the cancer at an early stage since detection can be focused on the gene mutation or polymorphism of the relevant type at the relevant site.

In addition to such cancer-related genes, detection of gene mutation or polymorphism of particular viruses or bacteria is known to be effective for diagnosis and treatment of particular infections caused by the particular viruses or bacteria.

For example, hepatitis C virus (HCV) which is responsible for the hepatitis C which constitute most of the transfusion hepatitis in Japan and which easily becomes chronic is known to exhibit gene polymorphism in the putative C gene region, and sensitivity to the therapeutic agent interferon is known to differ depending on the base sequence of this region. In view of such situation, an adequate diagnosis and treatment should be enabled if the patient could be determined for the suffering from complex infection, and if so, if the particular virus strain could be detected, identified, or quantitated for its mutation or polymorphism of the gene.

In the case of hepatitis B virus (HBV) which is responsible for the hepatitis B, the virus can be classified by the gene polymorphism in the base sequence of the pre-C region, and the polymorphism in this region has been reported to result in the difference in pathology and prognosis. When the risk of complex infection is taken into consideration, identification or quantitation of virus strain which may be present in a minute amount by utilizing the gene polymorphism is required for adequate diagnosis and treatment.

In the case of bacterial infection, there is also a risk that a minute amount of drug-resistant bacterium is present among the major amount of the drug-sensitive bacterium. Such drug-resistant bacterium is generally detected by utilizing the drug-resistant gene. A bacterium may acquire drug resistance through substitution of one base in a particular gene, and a reliable, rapid detection of the drug-resistant bacterium of a minute amount with such slight mutation is quite important in diagnosing and treating the bacterial infection.

In the case of bone marrow transplantation, it is quite important to judge whether the blood cells of the recipient has been fully replaced with the blood cells of the donor in deciding whether the transplantation has succeeded or failed, and in monitoring the occurrence and degree of the rejection. If there is a difference in the gene between the donor and the recipient through polymorphism, the remaining recipient cells can be detected at a high efficiency by utilizing such difference. For example, when HLA-DR of HLA (human leukocyte antigen) is matched in the transplantation and some difference in the HLA-DP is present between the donor and the recipient due to polymorphism, take of the transplanted cell as well as occurrence and degree of the rejection can be monitored by utilizing such polymorphism of the HLA-DP gene. In view of such situation, quick detection and identification at a high sensitivity of the gene of the recipient exhibiting gene polymorphism from the gene of the donor blood cell which is present in excessive amount is required. Furthermore, if the gene newly introduced by gene therapy and expression of mRNA from the gene could be distinguished and detected from the gene of the host and its expression, progress and effectivity of the gene therapy can be monitored.

In view of the needs as described above, various methods have been investigated and proposed for the detection and identification of the mutation and polymorphism of the gene.

An exemplary such process is dot blot hybridization using an oligonucleotide (PCR Methods and Applications, 1, 297, (1992)). In this process, hybridization with oligonucleotide probes which are specific (complementary) to the mutant or polymorphic base sequence of the gene in the sample DNA (amplification product) is allowed to take place, and the gene mutation or polymorphism is detected on the bases of the probe which underwent the hybridization. This process, however, requires a precise control of the conditions of the hybridization and washing, and as a consequence, the process is associated with the drawbacks of handling inconvenience and prolonged reaction period.

A process is also proposed wherein the mutant or polymorphic base sequence in the analyte nucleic acid is gene amplified by PCR using primers capable specifically amplifying such sequence, and the gene mutation or polymorphism is detected by the presence/absence of the amplification product (Nature, 324, 163 (1986)). This process, however, is far from being practical since this process requires PCR gene amplification steps of the number the same as the type of the primers, and the process scheme is quite complicated. The level of the reaction specificity required in this process is also extremely severe.

For the genes wherein the site of gene variation is rather limited as in the case of cancer-related k-ras gene, a detection process is proposed wherein the primers used in the PCR is altered in the base sequence near their 3' terminals such that a particular type of restriction enzyme-recognizing sequence is generated upon amplification of the normal gene (Oncogene, 6, 1079 (1991)). In this process, the amplification product is cleaved by using restriction enzymes after the amplification of the analyte nucleic acid to judge whether the restriction enzyme-recognizing sequence is cleaved to thereby detect the nucleic acid including the gene mutation or polymorphism. This process is also complicated and time-consuming since a strict control is required in the selection of the reaction conditions, in the handling of the restriction enzymes, and the like.

A process recently proposed by J. C. Nicolas et al. is the process conducted by preparing a labeled standard DNA by introducing biotin label and FITC label to each strand of the double stranded nucleic acid fragment including the target region whose mutation or polymorphism is to be detected; adding an excessive amount of the sample DNA including the non-labeled nucleic acid fragment of the same region as the standard DNA to the labeled standard DNA to thereby allow competitive hybridization to take place; and measuring the change in the amount of the initially added labeled standard DNA to judge the presence/absence of the fragment including the base sequence the same as the labeled standard DNA (EP-A 362042, Anal. Biochem. 205, 193 (1992)). This process has enabled a convenient, rapid detection of the gene mutation and polymorphism as well as typing of the gene types.

The process of Nicolas et al. is capable of detecting the mutation and the polymorphism when the mutation or the polymorphism is present on one of the alleles. However, the detection is difficult when only minute amount of the mutant or polymorphic gene is present in a large amount of the normal gene, or when the type of the mutation or the polymorphism is not identified.

The inventors of the present invention have also proposed a similar process, but which is reverse to the process of Nicolas et al., wherein an excessive amount of non-labeled standard DNA is added to the labeled sample DNA to allow for the competitive to take place. This process was capable of detecting the mutation or polymorphism irrespective of the type of the nucleic acid, and this process was also capable of readily calculating the percentage of the mutant or polymorphic sequence. (This process is hereinafter abbreviated as PCR-PHFA. PCT/JP94/01106; WO95/02068; Nucl. Acids. Res. 22, 1541 (1994)). This PCR-PHFA process enabled detection of the mutant or polymorphic gene of a minute amount contained in the normal gene of a large amount, whose detection has been difficult by the process of Nicolas et al. Identification of the type of the gene mutation or polymorphism and quantitation of the mutant or polymorphic gene was difficult even when such PCR-PHFA process were employed.

Various processes other than those described above have been proposed for the detection, identification, and quantitation of the mutation and polymorphism in the nucleic acid. In spite of such efforts, none of such processes have been put into actual use due to insufficiency in precision, detection speed, or handling convenience.

### BRIEF DESCRIPTION OF THE DRAWINGS

FIG. 1 is a schematic view explaining the nucleic acid assay process of the present invention.
FIG. 2 is a graph showing theoretical percentage (index value) of the labeled standard DNA that rehybridizes in the competitive hybridization in relation to the content of the target DNA.
FIG. 3 is a graph showing percentage (index value) of the labeled standard DNA that rehybridized in the competitive hybridization of Example 1 in relation to the content of the target DNA.

### SUMMARY OF THE INVENTION

The present invention has been completed in view of the situation as described above, and an object of the present invention is to provide a highly accurate nucleic acid assay process which is capable of rapidly, reliably and conveniently identifying, or identifying and quantitating the nucleic acid which includes mutation or polymorphism of the gene and which is present in a minute amount among the normal gene. Such identification or quantitation, which had been difficult to carry out by the conventional process, should be favorable for use in diagnosis and treatment of cancer at an early stage, diagnosis or virus or bacterial infections, judgment of the success/failure of bone marrow transplantation, and the like. It is also an object of the present invention to provide an assay kit therefor.

The inventors of the present invention have made an intensive study to accomplish the object as described above, and completed the present invention after finding that, when the nucleic acid is assayed by amplifying a particular region of the analyte nucleic acid in the specimen to prepare a double stranded sample DNA; adding an excessive amount of said sample DNA to a labeled standard DNA comprising a double stranded nucleic acid having a site capable of binding to a solid support on one strand and a detectable label on the other strand to allow competitive hybridization to take place; and detecting the rehybridized labeled standard DNA by utilizing said detectable label and said site capable of binding to a solid support to thereby evaluate the degree of exchange of the complementary strands that took place between said sample DNA and said labeled standard DNA for the detection of the target DNA which is the same as said labeled standard DNA and which is present in said sample DNA; a detection limit for the target DNA which is the same as said labeled standard DNA and which is present in said sample DNA may be preliminarily selected, and excessiveness of said sample DNA added to said labeled standard DNA in the competitive hybridization may be selected in accordance with the thus selected detection limit. By conducing the assay by such process, the target DNA which is the same as the labeled standard DNA and which is present in the sample DNA can be reliably detected even when such DNA is present at an extremely minute amount. In addition, the target DNA which is the same as the labeled standard DNA can be quantitated on the bases of the calibration curve theoretically derived from the excessiveness of the sample DNA. Furthermore, the nucleic acid including gene mutation or polymorphism which is present at a minute amount in the normal gene can be detected, identified, or even quantitated at a high precision by labeling the mutant nucleic acid or polymorphic nucleic acid and using the thus labeled mutant or polymorphic nucleic acid for the labeled standard DNA.

In the present invention, the assay is conducted by adding an excessive amount of non-labeled sample DNA to the labeled standard DNA as in the case of the process of Nicolas et al. to promote competitive hybridization, and measuring the resulting hybridizate for the label intensity to evaluate the extent to which the labeled standard DNA has been diluted. In this process, the type of the target DNA which is the same as the labeled standard DNA and which may be present in the sample DNA (i.e. the type of the nucleic acid including the gene mutation or polymorphism) is estimated and the detection limit for the target DNA including the gene mutation or polymorphism is selected; and the sample DNA of certain excessive amount is added to the labeled standard DNA which has been selected according to the estimated mutation and polymorphism. The detection limit is selected by estimating the extent of the dilution of the labeled standard DNA, and the resulting decrease in the label intensity of the hybridizate after the reaction, and the excessiveness of the sample DNA is determined on the bases of the thus selected detection limit. It is the sample DNA of such excessiveness that is added to the labeled standard DNA for competitive hybridization, and the label intensity of the hybridizate is thereafter measured. The detection is thereby conducted at the detection sensitivity within the optimal region corresponding to the content of the target DNA. As a consequence, the nucleic acid including the gene mutation or polymorphism of a minute amount is reliably detected and identified. Furthermore, since the detection is carried out at a detection sensitivity within the optimal range, the label intensity measured exhibits significant difference even when the content of the target DNA is minute, and quantitation of the nucleic acid including the gene mutation or polymorphism is thereby enabled.

Accordingly, the present invention provides a nucleic acid assay process comprising the steps of amplifying a particular region of the analyte nucleic acid in the specimen to prepare a double stranded sample DNA; adding an excessive amount of said sample DNA to a labeled standard DNA comprising a double stranded nucleic acid having a site capable of binding to a solid support on one strand and a detectable label on the other strand to allow competitive hybridization to take place; and detecting the rehybridized labeled standard DNA by utilizing said detectable label and said site capable of binding to a solid support to thereby evaluate the degree of exchange of the complementary strands between said sample DNA and said labeled standard DNA for detecting the target DNA which is the same as said labeled standard DNA and which is present in said sample DNA;
characterized in that
a detection limit for the target DNA which is the same as said labeled standard DNA and which is present in said sample DNA is preliminarily selected, and excessiveness of said sample DNA added to said labeled standard DNA in the competitive hybridization is selected in accordance with the thus selected detection limit.

The present invention also provides a nucleic acid assay kit for assaying a nucleic acid in accordance with the assay process as described above which is characterized by comprising a labeled standard DNA comprising a double stranded nucleic acid having a site capable of binding to a solid support on one strand and a detectable label on the other strand.

### BEST MODE FOR CARRYING OUT THE INVENTION

Next, the present invention is described in further detail.

In the assay process of the present invention, an excessive amount of the non-labeled sample DNA is added to the labeled standard DNA as in the case of the process of Nicolas et al. to promote competitive hybridization, and the resulting hybridized labeled standard DNA is detected to measure the extent of the exchange of complementary strands between the sample DNA and the labeled standard DNA. In detecting the target DNA which is the same as the labeled standard DNA and which is present in the sample DNA, a detection limit is preliminarily selected for the target DNA which is the same as the labeled standard DNA and which is present in the sample DNA, and the excessiveness of the sample DNA added to the labeled standard DNA in the competitive hybridization is determined on the bases of the thus selected detection limit. A reliable detection of the target DNA which is the same as the labeled standard DNA is thereby enabled even when the target DNA is present in a minute amount, and quantitation of the target DNA is also enabled.

The principle of the assay is described in the following. In the process of Nicolas et al., an excessive amount of the sample DNA is merely added to the labeled standard DNA comprising a double stranded nucleic acid having a site capable of binding to a solid support on one strand and a detectable label on the other strand; and the reaction mixture is annealed to promote competitive hybridization; and the reaction product is trapped on the solid support to measure the label intensity to thereby measure the rehybridized labeled standard DNA. The presence/absence of the nucleic acid fragment including the base sequence the same as the labeled standard DNA is thereby determined.

However, when the content of the target DNA which is the same as the labeled standard DNA is only 1%, and the assay is conducted at the excessiveness of the sample DNA of 10, the dilution of the labeled standard DNA is 10/11, and the labeled standard DNA is not substantially diluted. Therefore, the DNA whose content is 1% is not detected.

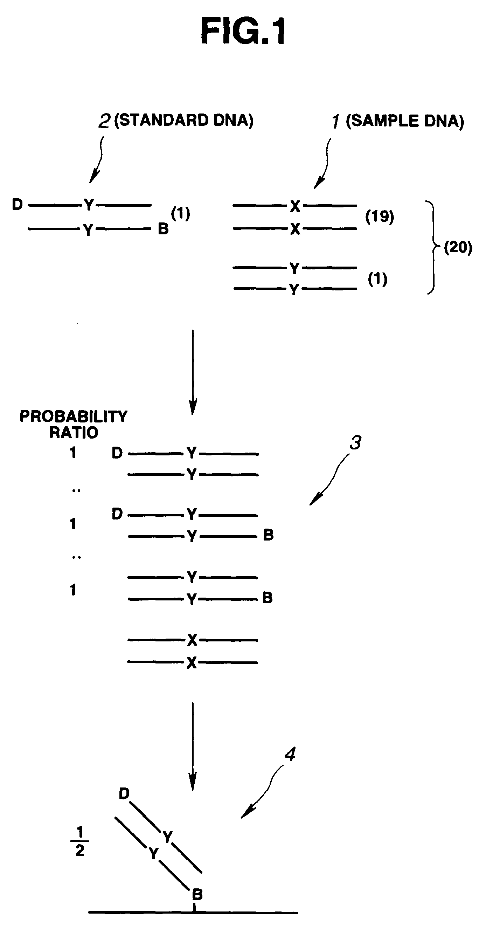

In view of such situation, in the present invention, a detection limit is preliminarily selected for the target DNA which is the same as the labeled standard DNA and which is present in the sample DNA. For example, when the target DNA whose content in the sample DNA is about 5% (1/20) is detected, the sample DNA1 is used at an excessiveness of, for example, 20 or more as shown in FIG. 1, and use of the DNA1 in such an amount means that 1/20 of the sample DNA1 is the target DNA whose base sequence is the same as the labeled standard DNA2. The labeled standard DNA2 is then diluted to 1/2, and the theoretical measurement is 1/2 of the initial measurement. A reliable detection of the target DNA which is the same as the labeled standard DNA2 and which is contained in the sample DNA1 is thereby realized. It should be noted that, even when the excessiveness of the sample DNA1 is 20 or more, the labeled standard DNA is theoretically not at all diluted if no DNA whose base sequence is the same as the labeled standard DNA2 is present in the sample DNA1. Therefore, the label intensity of the case wherein the content of the target DNA is 5% is 1/2 of the label intensity of the case wherein the target DNA is absent.

In a similar manner, when sample DNA of the amount 40 times in excess of the labeled standard DNA is added, and the test sample contains the target DNA including the base sequence the same as the labeled standard DNA in an amount of 2.5% (1/40), the proportion of the initial labeled standard DNA rehybridized will be 1/2, and the label intensity will also be 1/2. As exemplified in this case, detection and identification of even smaller minute amount of the target DNA having the base sequence the same as the labeled standard DNA is enabled by increasing the excessiveness of the sample DNA in relation to the labeled standard DNA. As described above, selection of the excessiveness of the sample DNA depending on the content in the sample DNA of the target DNA having the base sequence the same as the labeled standard DNA enables a reliable detection and identification of the target DNA even if the content of the target DNA was extremely low.

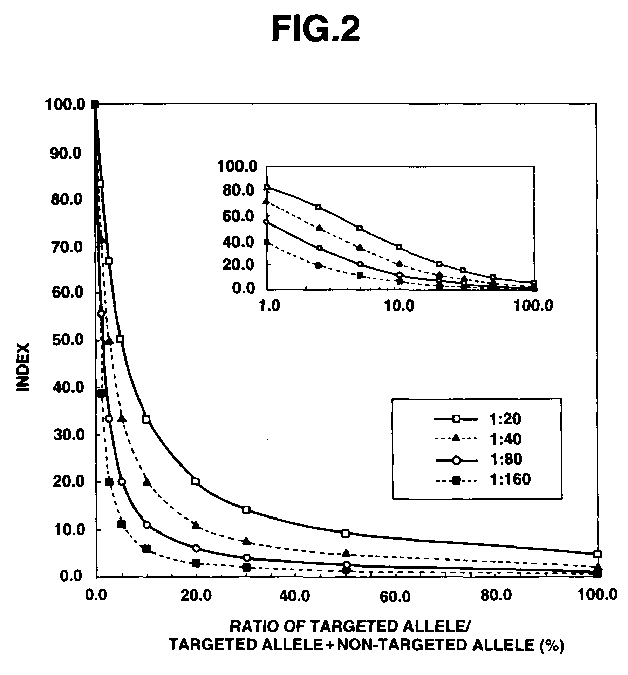

The theoretical influence on the proportion of the initial labeled standard DNA rehybridized of the excessiveness of the sample DNA in relation to the labeled standard DNA and the content in the sample DNA of the target DNA having the base sequence the same as the labeled standard DNA is calculated and shown in Table 1, below. FIG. 2 is the graphical representation of the Table 1.

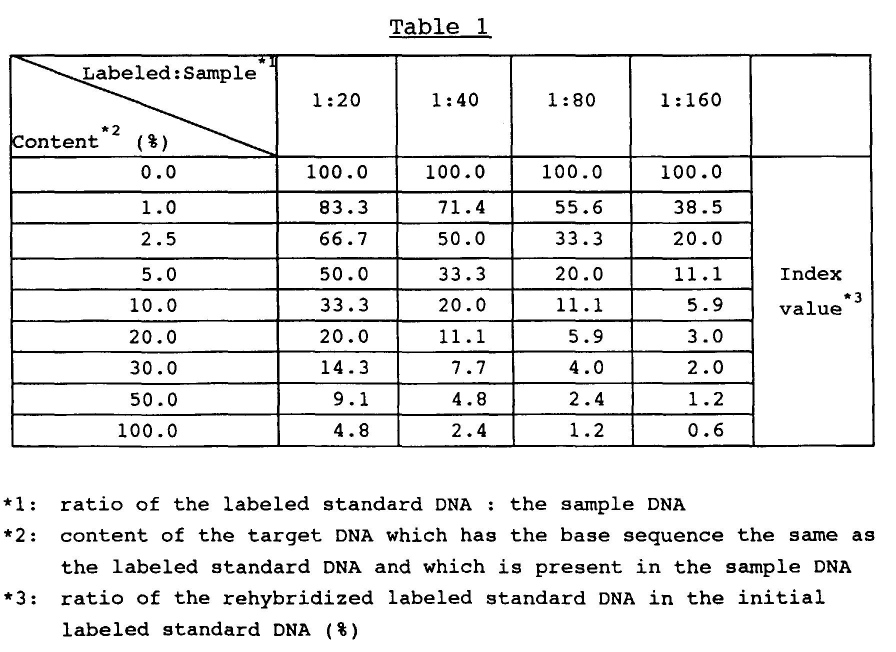

As shown in the theoretical calculation of Table 1 and FIG. 2, when the ratio of the labeled standard DNA : the sample DNA is 1:20, the index value is 50 when the content of the target DNA having the base sequence the same as the labeled standard DNA is 5%, and on the other hand, when the ratio of the labeled standard DNA : the sample DNA is 1:160, the index value is less than 50 when the content of the target DNA having the base sequence the same as the labeled standard DNA is 1%. As exemplified by these cases, the target DNA having the base sequence the same as the labeled standard DNA can be detected and identified by increasing the excessiveness of the sample DNA in relation to the labeled standard DNA even if the content of the target DNA is low. In addition, when the competitive hybridization is conducted at an adequate excessively, content of the target DNA having the base sequence the same as the labeled standard DNA can be theoretically calculated from the excessiveness and the obtained index value even if the content of the target DNA is low.

As described above, the assay process of the present invention is capable of reliably detecting, identifying and even quantitating the target DNA having the base sequence the same as the labeled standard DNA present in the sample DNA even if the content of the target DNA is quite minute, and such assay of high precision is enabled by preliminarily selecting the detection limit for the target DNA having the base sequence the same as the labeled standard DNA present in the sample DNA, determining the excessiveness of the sample DNA added to the labeled standard DNA depending on the selected detection limit, and adding the sample DNA of adequate excessiveness to the labeled standard DNA to thereby promote the competitive hybridization.

In carrying out the assay process of the present invention, the particular region of the analyte nucleic acid in the specimen is first amplified to prepare the double stranded sample DNA. Exemplary analyte nucleic acids are cancer-related genes, genes related to genetic diseases, virus genes, bacterial genes and polymorphic genes from the host.

The term, polymorphic genes from the host as used herein designates genes exhibiting polymorphism which have no direct relationship with the cause of a disease, and such genes include genes related to HLA and blood types. Although such genes are usually located on the chromosome of the host, there are cases wherein such gene is located on mitochondria. Exemplary specimens which contain such analyte nucleic acid are pathogens such as a bacterium and a virus; blood, saliva, section of tissue lesion and the like separated from a living body; and excreta such as feces and urine. In the case of prenatal diagnosis, cells of fetus in the amniotic fluid or a part of the divided ovule in the test tube may be used for the specimen. The specimens may be preliminarily subjected to cytolysis by treating the specimen using an enzyme, heat or a surfactant, ultrasonication, or a combination thereof directly or after optional concentration by precipitation through a procedure like centrifugation. Such cytolysis is effected for the purpose of exposing the DNA from the target tissue. In practice, the cytolysis may be carried out in accordance with a known procedure such as the one described in PCR PROTOCOLS, Academic Press Inc., p14, p352 (1990) or other documents. The specimen may preferably contain the DNA in a total amount of about 1 to 100 µg although the DNA in an amount of less than 1 µg is well amplifiable.

The gene amplification process used in amplifying the analyte nucleic acid in the specimen for the preparation of the sample DNA is not limited to any particular process, and such amplification may be carried out by using gene amplification primers or by chemical synthesis or enzymatically linking chemically synthesized partial sequences. In preparing the sample DNA, the thus amplified DNA may be mass-produced by incorporating the DNA in a vector selected from plasmid vectors, phage vectors and chimeric vectors derived from a plasmid and a phage and introducing the vector in a propagatable host such as a bacterium such as Escherichia coli or Bacillus subtilis, or yeast (Saccharomyces cerevisiae).

Exemplary preferable process using gene amplification primers are PCR (Polymerase Chain Reaction), LCR (Ligase Chain Reaction), 3SR (Self-sustained Sequence Replication), SDA (Strand Displacement Amplification), and the like (Manak, DNA Probes, 2nd Edition, p 255-291, Stockton Press (1993)), and the most preferred is PCR.

In the case of PCR, the primers used for the amplification of the analyte nucleic acid undergo primer extension reaction when the analyte nucleic acid is present, and the gene amplification is thereby accomplished. The primer extension is promoted by allowing 4 or 5 types of nucleotide triphosphates (deoxy adenosine triphosphate, deoxy guanosine triphosphate, deoxy cytidine triphosphate, and thymidine triphosphate or deoxy uridine triphosphate, the mixture of which is often called dNTP) to be incorporated into the primer as the substrates.

In the extension of the nucleic acid, amplification reagents including the unit nucleic acid as described above and nucleic acid extending enzyme are usually employed. The nucleic acid extending enzyme may comprise any DNA polymerase such as E. coli DNA polymerase I, Klenow fragment of E. coli DNA polymerase I, T4 DNA polymerase, or the like. The most preferred are thermally stable DNA polymerases such as Taq DNA polymerase, Tth DNA polymerase, Vent DNA polymerase, and the like, and use of such thermally stable DNA polymerase enables automatic repetition of the amplification cycle with no need of supplementing the fresh enzyme in each cycle as well as use of an annealing temperature in the range of from 50 to 60°C to realize an improved specificity in the recognition of the target sequence by the primer. A rapid gene amplification of high specificity is thereby realized. (For further detail, see Japanese Patent Application Laid-Open Nos. 1-314965 and 1-252300.)

In this reaction, an oil may be added to the reaction solution for the purpose of preventing evaporation of the moisture content. Any oil can be used as long as the oil separates from the water and has a specific gravity lower than the water, and typical oils include silicone oil, mineral oil, and the like. Such oil is unnecessary in some gene amplification apparatus, and the primer extension reaction may also be carried out in such an apparatus.

By repeating the extension reaction using the nucleic acid amplification primers as described above, the nucleic acids in the specimen can be amplified at a high efficiency to enable the production of the sample DNA in a large amount. It should be noted that the specific conditions and the like for the gene amplification may be determined in accordance with the known methods described in various references such as Jikken Igaku (Experimental Medicine), Yodosha, 8, No. 9 (1990), and PCR Technology, Stockton Press (1989).

Next, the sample DNA obtained by the gene amplification as described above of an excessive amount is added to the labeled standard DNA for the competitive hybridization.

The labeled standard DNA is a double stranded nucleic acid which has a sequence the same as the target DNA in the sample DNA and which has a site capable of binding to a solid support on one strand and a detectable label on the other strand.

Such detectable label may be either a radioactive substance or a non-radioactive substance, and the label is preferably a non-radioactive substance. Of the nonradioactive substances which may be used for the label, exemplary substances which may be used as a direct label are fluorescent substances [such as fluorescein derivatives (fluorescein isothiocyanate etc.), rhodamine and its derivatives (tetramethylrhodamine isothiocyanate etc.)], chemiluminescent substances (such as acridine), and the like (see Japanese Patent Application Laid-Open No. 1-252300). The label may also be indirectly detected by using a substance which specifically binds to the label, and examples of such label include biotin, ligands, nucleic acids and proteins of particular types, haptens and the like. For example, biotin may be used with avidin or streptavidin which specifically binds to the biotin, and the hapten may be used with an antibody which specifically binds to the hapten. The ligand may be used with a receptor, and the nucleic acid or the protein of particular type may be used with a nucleic acid or a nucleic acid-binding protein which specifically binds to such nucleic acid or with a protein which has affinity to such protein. Exemplary such haptens include compounds having 2,4-dinitrophenyl group and digoxigenin, and the above-mentioned biotin and fluorescent substances can also be used as a hapten. It should be noted that the detectable label may be the same as the region capable of binding to a solid support.

The labeled standard DNA may be prepared as described below. When the target DNA is a mutant nucleic acid or a polymorphic nucleic acid, the labeled standard DNA may be prepared, for example, by preparing a nucleic acid which includes the gene mutation or polymorphism in the particular region of the analyte nucleic acid, and amplifying this nucleic acid by using a primer having incorporated therein a site capable of binding to a solid support and a primer having incorporated therein a detectable label. In this case, the analyte nucleic acid may not necessarily include the mutation or polymorphism, and the labeled standard DNA including the mutation or polymorphism may be prepared by conducting the gene amplification using a pair of primers whose base sequence includes such mutation or polymorphism.

Instead of the gene amplification, the labeled standard DNA may be prepared by enzymatically cleaving a natural gene by using restriction enzymes. The labeled standard DNA may also be mass-produced by amplifying the normal nucleic acid, and then incorporating the amplified nucleic acid in a vector selected from plasmid vectors, phage vectors and chimeric vectors derived from a plasmid and a phage and introducing the vector in a propagatable host such as a bacterium such as Escherichia coli and Bacillus subtilis, and yeast (Saccharomyces cerevisiae) (gene cloning). In some cases, the labeled standard DNA may be produced by chemical synthesis. Typical chemical synthesis processes are triester method and phosphite method, wherein the labeled standard DNA may be produced by mass-producing the single strand DNA in a conventional automatic synthesizer (for example, model 392 manufactured by Applied Biosystems) by using liquid phase method or solid phase synthesis method using an insoluble support, and annealing the single strand DNA to produce the double stranded DNA. In this case, amino group may be introduced in the 5' terminal of the resulting double stranded DNA (see Japanese Patent Application Laid-Open No. 59-93100), and an appropriate label may be introduced in the amino group (see Japanese Patent Application Laid-Open Nos. 59-204200 and 59-148798) to thereby prepare the labeled standard DNA.

In the assay process of the present invention, competitive hybridization is conducted by adding the sample DNA of an excessive amount to the thus prepared labeled standard DNA, and as described above, a detection limit is preliminarily selected for the target DNA in the sample DNA, and excessiveness of the sample DNA is also determined in accordance with the thus selected detection limit. The detection limit of the target DNA in the sample DNA may be appropriately selected depending on the type of the analyte nucleic acid and the target DNA, and the detection limit is generally up to 10% in the case of acquired mutation or polymorphism of a cancer-related gene such as k-ras gene, N-ras gene, p53 gene, BRCA1 gene, BRCA2 gene, or APC gene; generally up to 10% in the case of mutation or polymorphism of a virus gene or bacterium; 50% in the case of mutation or polymorphism in chromosomal DNA; and up to 1% in the case of mitochodoria gene.

As described above, the excessiveness of the sample DNA is determined in accordance with the detection limit. The excessiveness of the sample DNA is not particularly limited, and the excessiveness may be selected such that the target DNA can be detected and identified after the competitive hybridization on the bases of the label intensity of the hybridizate. In practice, an adequate detection limit may be selected in accordance with the assay method of the label intensity and the like, and the excessiveness may then be selected by taking the theoretical index value as described above into consideration. For example, when the target DNA is detected and identified in the assay, the excessiveness may be selected so that a large difference is exhibited between the label intensity in the presence of the target DNA and the label intensity in the absence of the target DNA to facilitate an easy judgment between the presence and absence of the target DNA. When the target DNA is quantitated in the assay, the excessiveness may be selected depending on the expected content of the target DNA so that a relatively large difference in the intensity is observed in the vicinity of the expected target DNA content and an effective calibration curve can be depicted.

The procedure of excessiveness determination is not particularly limited, and the excessiveness may be typically determined such that, when the detection limit selected for the target DNA which is the same as the labeled standard DNA and which is present in the sample DNA is A/B, the excessiveness of the sample DNA may be at least B/A, preferably 1.5B/A, and more preferably 2A/B to 1000A/B. The sample DNA may be preliminarily quantitated for the amount of DNA (total content) in the sample DNA by such means electrophoresis, UV irradiation or absorption so that an adequate amount of DNA may be added in accordance with the excessiveness.

In carrying out the competitive hybridization by adding the sample DNA of an excessive amount to the labeled standard DNA, the sample DNA and the labeled standard DNA should be denatured in the first place, and the denaturation is preferably accomplished by thermal or alkaline denaturation. The sample DNA and the labeled standard DNA may be mixed either immediately before the denaturation or after the denaturation.

In the competitive hybridization, the reaction solution should be adjusted to thereby optimize the salt concentration which depends on the length of the nucleic acid. In the hybridization, SSC (20 x SSC: 3M sodium chloride, 0.3M sodium citrate) and SSPE (20 x SSPE: 3.6M sodium chloride, 0.2M sodium phosphate, 2mM EDTA) are generally used for the salt concentration adjustment, and such solution can also be used in the assay process of the present invention after diluting to an appropriate concentration and after optional supplementation with an organic solvent such as dimethylsulfoxide (DMSO) and dimethylformamide (DMF).

The competitive hybridization may be accomplished by mixing the sample DNA and the labeled standard DNA which have been denatured as described above, and gradually reducing the temperature from the high temperature. The temperature conditions may be adequately optimized according to the length and sequence of the DNA to be hybridized and the difference between the denatured base sequence and the normal base sequence. The temperature conditions, however, are generally such that the temperature is reduced from 98°C to 50°C at a rate of 1°C per 3 to 10 minutes, and more preferably, such that the temperature is reduced from 98°C to 70°C at a rate of 1°C per 10 minutes.

Next, the product of the competitive hybridization is measured on the bases of the principle of ED-PCR (see, for example, Japanese Patent Application Laid-Open Nos. 1-314965 and 1-252300; J. Clin. Microbiol. 30, 1728 (1992)). In the ED-PCR, the presence of the double stranded nucleic acid is indicated as a signal only when the double stranded nucleic acid has different labels on each strand (The label may not necessarily be different). Accordingly, the product of the competitive hybridization will exhibit lower signal intensity with the increase in the occurrence of the strand exchange between the sample DNA and the labeled standard DNA. In other words, the signal intensity decreases with the increase in the analyte nucleic acid which has the base sequence the same as the labeled standard DNA in its particular region.

As described above, the hybridizate is measured and the presence/absence or content of the nucleic acid having gene mutation or polymorphism is determined from the results of the measurement. The measurement may be carried out by a conventional procedure selected in accordance with the label used. For example, when the label is a radioisotope, intensity of the radioactivity may be measured with a scintillation counter, and when the label is a fluorescent substance, intensity of the fluorescence may be measured with a fluorophotometer (see Japanese Patent Application Laid-Open No. 1-252300).

On the other hand, when the labeled standard DNA has incorporated therein a label other than the directly detectable label, a reagent adapted for indirect measurement of the label is used. When the label is biotin, a complex of avidin or streptavidin with an enzyme is used, and when the label is a hapten, an antibody-enzyme complex which is a complex between an antibody which specifically binds to the hapten and an enzyme, or a substrate for such enzyme is used. By using such reagent, the label undergoes a reaction with the reagent to produce a component which can be detected by means of color development or fluorescence. The enzyme and the substrate which may be used in such reagent are as described below. When the enzyme is β-D-galactosidase, the substrate may be 2-nitrophenol-β-D-galactoside or 4-methylumbelliphelyl-β-D-galactoside. When the enzyme is peroxidase, the substrate may be 3-(4-hydroxyphenyl)propionic acid, 3,3',5,5'-tetramethylbenzidine, 1,2-phenylenediamine, or the like. When the enzyme is alkaline phosphatase, the substrate may be 4-methylumbelliphelyl phosphate, NADP, 4-nitrophenyl phosphate, or the like. When the enzyme is glucose-6-phosphate dehydrogenase, the substrate may be glucose, NAD, or the like. When the enzyme is an alcohol dehydrogenase, the substrate may be ethanol, NAD, or the like.

It should be noted that the measurement of the hybridizate is carried out by trapping the hybridizate on a solid support. The solid support used should be a support which is capable of specifically binding to the solid support-binding site of the primer, and the typical such solid support is a well of a microtiter plate which has been pretreated to facilitate the specific binding to the solid support-binding site of the primer.

When the sample DNA has no base sequence which is the same as the labeled standard DNA, the exchange rate of complementary strands between the labeled standard DNA and the sample DNA is low, and the label intensity is not significantly reduced. In contrast, when the base sequence which is the same as the labeled standard DNA is present in the particular region of the analyte nucleic acid, exchange of complementary strands between the labeled standard DNA and the sample DNA occurs at a higher frequency, and the label intensity is reduced. In view of such situation, in the assay process of the present invention, a detection limit for the target DNA such as mutant or polymorphic nucleic acid in the sample DNA is preliminarily selected corresponding to the purpose of the assay, and amount (excessiveness) of the sample DNA added is determined in accordance with the thus selected detection limit, and the competitive hybridization is thereafter carried out. As a consequence, the label intensity markedly decreases when the base sequence the same as the labeled standard DNA is present in the particular region of the analyte nucleic acid even if the content of such base sequence is quite low, and a reliable detection and identification is thereby enabled, and also, the label intensity significantly varies with the content of the nucleic acid enabling a convenient quantitation.

Next, the assay kit according to the present invention is described. The assay kit of the present invention is a kit for detecting, identifying and quantitating the nucleic acid which has a gene mutation or polymorphism in accordance with the assay process of the present invention, and the kit includes a labeled standard DNA comprising a double stranded nucleic acid having a site capable of binding to a solid support on one strand and a detectable label on the other strand. In such a case, the detectable label and the region capable of binding to the solid support in the labeled standard DNA may be the same as those described in relation to the nucleic acid assay process of the present invention, and the labeled standard DNA may be prepared by the procedure as described for the nucleic acid assay process of the present invention.

The assay kit of the present invention is used in accordance with the assay process of the present invention as described above by preparing the sample DNA from the specimen which has been optionally subjected to a pretreatment such as cell lysis, adding an excessive amount of the sample DNA to the labeled standard DNA to allow for the competitive hybridization to take place; and trapping the hybridizate on the solid support to thereby measure the degree of exchange of the complementary strands between the DNAs of both types. In such a case, those known in the art may be used for the pair of primers for amplifying the particular region of the analyte nucleic acid in the specimen, the reagents for amplifying the sample DNA, and the support for trapping the hybridizate, and the kit for detecting, identifying or quantitating the nucleic acid which has mutation or polymorphism in a particular region of the analyte nucleic acid may be completed by incorporating such components.

The assay kit of the present invention may also contain a reagent for cytolysis used in the pretreatment of the specimen, a washing solution for washing the amplification reaction product, an oil for preventing evaporation of the moisture content of the reaction solution, reagents for indirect detection of the label, and the like as described in relation to the nucleic acid assay process of the present invention, and the assay kit of the present invention may be completed by incorporating such components in suitable combinations.

The present invention is described in further detail by referring to the Examples and Comparative Examples which by no means limit the scope of the present invention.

### [Example 1]

A model for detecting mutation or polymorphism wherein the gene is substituted at one base is described.

### Primers

A pair of primers (k-ras-5n, k-ras-3) of the base sequence fully complementary to human chromosomal k-ras gene and a pair of primers (k-ras-5m, k-ras-3) including a mismatch of one base were used. The primers are shown below.
k-ras-5n
   SEQ ID No: 1
   5'-TATAAACTTGTGGTAGTTGGAGCT-3'
k-ras-5m (the substituted base is underscored)
   SEQ ID No: 2
   5'-TATAAACTTGTGGTAGTTGGACCT-3'
k-ras-3
   SEQ ID No: 3
   5'-TATCGTCAAGGCACTCTTGCC-3'

### Preparation of labeled standard DNA

Gene amplification by PCR was conducted by using 100 ng of k-ras gene from human normal chromosome for the template, and Bio-k-ras-5m (100 ng) having introduced biotin therein and DNP-k-ras-3 (100 ng) having introduced DNP for the primers in the presence of 200 µM of dNTP in a reaction solution containing 100 µl of 10 mM Tris-HCl buffer solution (pH 8.3), 50 mM KCl, 1.5 mM MgCl₂, 0.001% gelatin, and 2 units of Ampli taq™ DNA polymerase. The PCR was performed by repeating 35 cycles of 30 sec. at 94°C, and 30 sec. at 56°C using Thermal Cycler PJ2000 (manufactured by Perkin Elmer). The reaction solution was electrophoresed on agarose gel to confirm the size of the amplified product and the amplification efficiency.

### Preparation of non-labeled DNA

The non-labeled DNA having the base sequence the same as the human chromosomal k-ras gene was amplified by repeating the PCR process as described above using NH₂-k-ras-5n and NH₂-k-ras-3 for the primers in the same reaction solution. The reaction solution was electrophoresed on agarose gel to confirm the size of the amplified product and the amplification rate.

The non-labeled DNA having the sequence which is the same as the human chromosomal k-ras gene except for one base was amplified by repeating the PCR process as described above using NH₂-k-ras-5m and NH₂-k-ras-3 for the primers in the same reaction solution. The reaction solution was evaluated as described above.

### Preparation of sample DNA

The thus produced non-labeled DNA having the base sequence the same as the normal gene and the thus produced non-labeled DNA having the sequence which is the same as the normal gene except for one base were mixed at various ratios to prepare non-labeled sample DNAs wherein the mutant gene is present at proportion of 0.0%, 1.0%, 2.5%, 10.0%, 20.0%, 30.0%, 50.0%, and 100.0% in the normal gene.

### Competitive hybridization

Next, the labeled standard DNA was mixed with various non-labeled sample DNAs at various ratios, and competitive hybridization was allowed to take place.

A system wherein the labeled standard DNA and the non-labeled sample DNA were at a mixing ratio of 1:20 (prepared by mixing the PCR reaction solutions at the ratio of 1 µl of the labeled standard DNA to 20 µl of the non-labeled sample DNA); and a system wherein the labeled standard DNA and the non-labeled sample DNA were at a mixing ratio of 1:40 (prepared by mixing the solutions at the ratio of 0.5 µl of the labeled standard DNA to 20 µl of the non-labeled sample DNA) were prepared. The mixed solution had a salt concentration of 3.3 x SSC (20 x SSC : 0.3M sodium citrate, pH 7.0, 3M sodium chloride), and the volume of the reaction solution was 30 µl. The solution was heated to 98°C for 10 minutes by using Programmable Thermal Controller PTC-100 (manufactured by MJ Research) for thermal denaturation of the DNA, and the reaction solution was then gradually cooled to 68°C at 1°C/10 min to promote the hybridization (competitive hybridization).

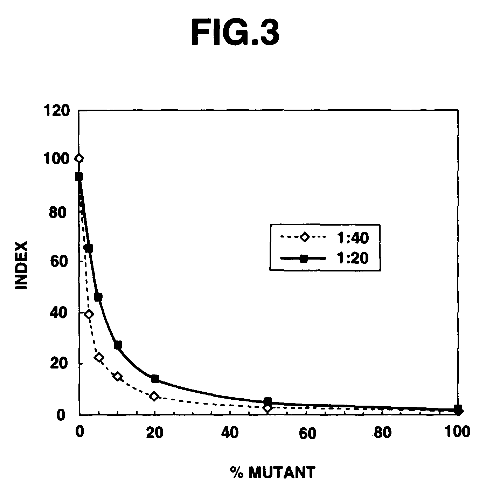

20 µl of the reaction solution was aliquoted, and the amount of the rehybridized labeled standard DNA was evaluated by means of ED-PCR to quantitatively determine the percentage of the rehybridized labeled standard DNA, i.e. the percentage (index value) of the rehybridized labeled standard DNA in the labeled standard DNA which had been present before the competitive hybridization. The results are shown in the graph of FIG. 3 wherein the index value is shown in relation to the content of the mutant gene.

As shown in the results of FIG. 3, when the labeled standard DNA and the non-labeled sample DNA were mixed at the ratio of 1:20, an index value of about 50 was obtained at the mutant gene content of about 5.0%, and the mutant gene was then sufficiently detectable. When the labeled standard DNA and the non-labeled sample DNA were mixed at the ratio of 1:40, an index value of about 50 was obtained at the mutant gene content of about 2.5%, and the mutant gene was then sufficiently detectable. It was then confirmed that the detection sensitivity actually increases with the increase in the amount (excessiveness) of the non-labeled sample DNA in relation to the labeled standard DNA.

It was also confirmed that there is a correlation between the index value and the mutant gene content, and that percentage of the mutant gene in the specimen (mutant gene content) can be quantitatively determined when the index value and the amount (excessiveness) of the non-labeled sample DNA in relation to the labeled standard DNA are known.

### [Example 2]

Labeled substituted standard DNAs of all substitution patterns wherein the twelfth codon of the human chromosomal k-ras gene is substituted at one base such that the substitution results in the amino acid substitution were prepared, and the procedure of Example 1 was repeated to thereby detect and identify the gene mutation as described below.

Substitutions of the twelfth codon GGT (Gly) of the normal k-ras gene at one base which result in the substitution of the amino acid include the following 6 substitutions: GCT (Ala), GTT (Val), GAT (Asp), CGT (Arg), AGT (Ser), and TGT (Cys).

### Preparation of plasmid

0.1 µg of normal human chromosomal DNA was gene amplified by PCR by using 100 ng each of the primers (K12-P1, K12-P2) as described below under the conditions of Example 1. The resulting fragment was introduced in the plasmid pBluescript (available from STRATAGENE), and the base sequence was confirmed. The resulting plasmid was designated plasmid pKR1.
K12-P1
   SEQ ID No: 4
   5'-GGCCTGCTGAAAATGACTGA-3'
K12-P2
   SEQ ID No: 5
   5'-TTGTTGGATCATATTCGTCC-3'

The 6 types of the mutant DNAs were prepared by oligonucleotide directed mutagenesis (see Shuntaro IKAWA, Supplementary volume of Jikken Igaku (Experimental Medicine), "Genetic Engineering Handbook", page 246) using the plasmid pKR1. The resulting plasmid (the standard DNA) was determined for its base sequence to confirm the production of the 6 types of standard DNAs having the desired base sequence.

### Primers

The primers used for the gene amplification had the base sequence as described below.
k-ras-43-5
   SEQ ID No: 6
   5'-AACTTGTGGTAGTTGGAGCT-3'
k-ras-43-3
   SEQ ID No: 7
   5'-AAGGCACTCTTGCCTACGCC-3'

### Preparation of labeled standard DNA

Gene amplification by PCR was conducted by repeating the procedure of Example 1 using 1 ng of the plasmid having incorporated therein one of the 7 genes (standard DNA) for the template, and labeled Bio-k-ras-43-5 (100 ng) and DNP-k-ras-43-3 (100 ng) prepared by labeling the above-described primers for the primers to prepare 7 types in total of the labeled standard DNAs.

### Preparation of non-labeled DNA

The non-labeled DNA was gene amplified by repeating the PCR procedure of Example 1 using 1 ng of the plasmid having incorporated therein one of the 7 genes (standard DNA) for the template, and labeled NH₂-k-ras-43-5 (100 ng) and NH₂-k-ras-43-3 (100 ng) for the primers to prepare 7 types in total of the non-labeled DNAs.

### Preparation of sample DNA

The thus produced non-labeled DNA each having one of the 6 mutant gene was mixed with the non-labeled DNA having the base sequence the same as the normal gene to prepare non-labeled sample DNAs each comprising 98% of the normal gene and 2% GCT mutant gene; 99% of the normal gene and 1% GCT mutant gene; 99% of the normal gene and 1% GTT mutant gene; 99% of the normal gene and 1% GAT mutant gene; 99% of the normal gene and 1% CGT mutant gene; 99% of the normal gene and 1% AGT mutant gene; 99% of the normal gene and 1% TGT mutant gene; and 98% of the normal gene and 1% GCT + 1% CGT mutant gene.

### Competitive hybridization

Competitive hybridization was conducted by using the PCR solution comprising 0.125 µl of the labeled standard DNA mixed with 20 µl (excessiveness, 160) of the non-labeled sample DNA. Other components of the reaction solution and the reaction conditions were as in the case of the Example 1. In this case, competitive hybridization for contrast purpose was conducted by using water instead of the non-labeled sample DNA.

20 µl of the reaction solution was aliquoted, and the amount of the rehybridized labeled standard DNA was evaluated by means of ED-PCR as the intensity of the developed color. The results are shown in Tables 2 and 3. The results are shown in Table 2 as the percentage (index value) when the intensity of the developed color in the case of the water was 100. The results are shown in Table 3 as the percentage (index value) when the intensity of the developed color in the case of the specimen ("normal") containing 100% of normal gene (GGT) was 100.

**Table 2**

| Labeled standard DNA | | Non-labeled sample DNA | | | | | | | | | |
|---|---|---|---|---|---|---|---|---|---|---|---|
| | | H₂O | normal | 2% GCT | 1% GCT | 1% GTT | 1% GAT | 1% CGT | 1% AGT | 1% TGT | 1% GCT + 1% CGT |
| 1 | GGT | 100.0 | 0.7 | 1.4 | 2.2 | 1.5 | 1.4 | 0.9 | 2.1 | 1.6 | 1.8 |
| 2 | GCT | 100.0 | 71.7 | 23.2 | 32.0 | 71.7 | 91.9 | 71.8 | 70.8 | 73.1 | 29.8 |
| 3 | GTT | 100.0 | 48.9 | 42.9 | 47.1 | 21.7 | 46.7 | 50.1 | 48.2 | 47.0 | 43.5 |
| 4 | GAT | 100.0 | 60.5 | 52.6 | 60.3 | 58.5 | 21.8 | 56.6 | 60.0 | 56.5 | 56.7 |
| 5 | CGT | 100.0 | 77.8 | 74.2 | 74.2 | 73.2 | 71.7 | 24.7 | 72.3 | 71.9 | 24.6 |
| 6 | AGT | 100.0 | 56.4 | 55.7 | 56.8 | 53.8 | 56.4 | 49.6 | 20.1 | 52.6 | 52.0 |
| 7 | TGT | 100.0 | 57.4 | 55.3 | 54.6 | 59.0 | 58.5 | 53.7 | 53.8 | 21.9 | 53.4 |

**Table 3**

| Labeled standard DNA | | Non-labeled sample DNA | | | | | | | | | |
|---|---|---|---|---|---|---|---|---|---|---|---|
| | | H₂O | normal | 2% GCT | 1% GCT | 1% GTT | 1% GAT | 1% CGT | 1% AGT | 1% TGT | 1% GCT + 1% CGT |
| 1 | GGT | - | - | - | - | - | - | - | - | - | - |
| 2 | GCT | - | 100 | 32 | 45 | 100 | 128 | 100 | 99 | 102 | 42 |
| 3 | GTT | - | 100 | 88 | 96 | 44 | 96 | 102 | 99 | 96 | 89 |
| 4 | GAT | - | 100 | 87 | 100 | 97 | 36 | 93 | 99 | 93 | 94 |
| 5 | CGT | - | 100 | 95 | 95 | 94 | 92 | 32 | 93 | 92 | 32 |
| 6 | AGT | - | 100 | 99 | 101 | 95 | 100 | 88 | 36 | 93 | 92 |
| 7 | TGT | - | 100 | 96 | 95 | 103 | 102 | 93 | 94 | 38 | 93 |

As shown in the results of Table 2, the specimens containing 2% or 1% of GCT exhibited significantly smaller index values for the labeled standard DNA (GGT) from the normal gene and for the labeled standard DNA from the GCT-mutant gene, revealing the presence of the GCT-mutant gene. Similarly, the specimens containing 1% GCT and 1% CGT exhibited significantly smaller index values for the labeled standard DNAs from the normal gene, the GCT-mutant gene, and the CGT-mutant gene, revealing the presence of the GCT-mutant gene and the CGT-mutant gene. Other specimens also exhibited significantly smaller index values for the labeled standard DNAs from the normal gene and the mutant gene of the corresponding type to confirm the presence in the non-labeled sample DNA specimen of the base sequence which is the same as the labeled standard DNA which exhibited the smaller index value.

As demonstrated above, the procedure as described above is capable of not only detecting the codon including the mutation or polymorphism of the gene in the specimen but also identifying the mutant or polymorphic base sequence.

As shown in Table 3, the index values of the specimens were in the range of 45 to 32, and it was then indicated that the content of the mutant gene is in the range of 1 to 2% on the bases of the theoretical index values shown in Table 1 and the calibration curve of FIG. 2, and the consistency with the actual content was confirmed. Therefore, the procedure as described above is capable of quantatively determining the content of the mutant or polymorphic gene.

### [Example 3]

Chromosomal k-ras gene was actually extracted from human cancer tissue to prepare specimens #1 to #4. The "normal" is the specimen comprising 100% of the normal gene. By using these genes for the templates, gene amplification by PCR was conducted by repeating the procedure of Example 2 to prepare the non-labeled sample DNAs. 20 µl of the thus prepared the non-labeled sample DNA was added to and mixed with 0.125 µl of the 7 types of the labeled standard DNAs as in the case of Example 2 (excessiveness, 160) to allow the competitive hybridization to take place. The index values were determined as in the case of the Example 2. The results are shown in Tables 4 and 5. The results are shown in Table 4 as the percentage (index value) when the intensity of the developed color in the case of the water was 100. The results are shown in Table 5 as the percentage (index value) when the intensity of the developed color in the case of the specimen ("normal") containing 100% of normal gene (GGT) was 100.

**Table 4**

| Labeled DNA | | H₂O | normal | specimen #1 | specimen #2 | specimen #3 | specimen #4 |
|---|---|---|---|---|---|---|---|
| 1 | GGT | 100.0 | 0.7 | 0.8 | 0.9 | 0.7 | 0.6 |
| 2 | GCT | 100.0 | 71.7 | 74.0 | 69.2 | 66.1 | 70.9 |
| 3 | GTT | 100.0 | 48.9 | 45.3 | 49.2 | 10.0 | 43.8 |
| 4 | GAT | 100.0 | 60.5 | 56.5 | 5.4 | 55.7 | 57.3 |
| 5 | CGT | 100.0 | 77.8 | 74.3 | 75.3 | 74.6 | 2.3 |
| 6 | AGT | 100.0 | 56.4 | 57.3 | 51.8 | 48.6 | 56.7 |
| 7 | TGT | 100.0 | 57.4 | 55.3 | 53.9 | 58.3 | 53.8 |

**Table 5**

| Labeled DNA | | H₂O | normal | specimen #1 | specimen #2 | specimen #3 | specimen #4 |
|---|---|---|---|---|---|---|---|
| 1 | GGT | - | - | - | - | - | - |
| 2 | GCT | - | 100.0 | 103.2 | 96.5 | 92.2 | 98.9 |
| 3 | GTT | - | 100.0 | 92.6 | 100.6 | 20.4 | 89.6 |
| 4 | GAT | - | 100.0 | 93.4 | 8.9 | 92.1 | 94.7 |
| 5 | CGT | - | 100.0 | 95.5 | 96.8 | 95.9 | 3.0 |
| 6 | AGT | - | 100.0 | 101.6 | 91.8 | 86.2 | 100.5 |
| 7 | TGT | - | 100.0 | 96.3 | 93.9 | 101.6 | 93.7 |

As shown in the results of Table 4, the specimen #1 exhibited small index value only for the labeled standard DNA (GGT) from the normal gene. Such pattern which is the pattern the same as the "normal" specimen revealed the absence of the mutant gene. The specimen #2 exhibited significantly smaller index values for the labeled standard DNAs from the GGT-normal gene and the GAT-mutant gene, revealing the presence of the GAT-mutant gene. In a similar manner, specimen #3 was found to contain the GTT-mutant gene, and specimen #4 was found to contain the CGT-mutant gene.

In addition, when the content of the mutant gene (content of the mutant gene or polymorphic gene in the normal gene) was determined from the index values shown in the Table 5 and the excessiveness (labeled standard DNA : non-labeled sample DNA = 1 : 160) on the bases of the theoretical index values shown in Table 1 and the calibration curve of FIG. 2, specimen #2 was estimated to have a GAT-mutant gene content of about 7%, specimen #3 was estimated to have a GTT-mutant gene content of about 2.5%, and specimen #4 was estimated to have a CGT-mutant gene content of about 20%.

### [Example 4]

Labeled substituted standard DNAs of all substitution patterns wherein the twelfth codon of the human chromosomal N-ras gene is substituted at one base such that the substitution results in the amino acid substitution were prepared, and the procedure of Example 1 was repeated to thereby detect and identify the gene mutation as described below.

Substitutions of the twelfth codon GGT (Gly) of the normal N-ras gene at one base which result in the substitution of the amino acid include the following 6 substitutions: AGT (Ser), CGT (Arg), TGT (Cys), GAT (Asp), GCT (Ala), and GTT (Val).

### Primers

The primers used for the gene amplification had the base sequence as described below.
N-12S
   SEQ ID No: 8
   5'-AACTGGTGGTGGTTGGAGCA-3'
N-12A
   SEQ ID No: 9
   5'-GATTGTCAGTGCGCTTTTCCC-3'

### Preparation of labeled standard DNA

1 pg/µl solutions of the fragments respectively corresponding to the wild (GGT) gene and the mutant genes (7 types in total) were prepared by using ras Mutant set, N-ras codon 12 manufactured by Takara. Gene amplification by PCR was conducted by repeating the procedure of Example 1 using 1 pg of the fragment for the template, and Bio-N-12A (100 ng) and DNP-N-12S (100 ng) prepared by labeling the above-described primers for the primers to prepare 7 types in total of the labeled standard DNAs.

### Preparation of non-labeled sample DNA

The non-labeled DNAs were prepared as described below. The fragments containing the mutant gene were respectively mixed with the fragment having the base sequence of the normal gene (GGT) to prepare 6 types of templates each containing a minute amount of the mutant gene: the templates comprising 99% of the normal gene and 1% AGT mutant gene; 99% of the normal gene and 1% CGT mutant gene; 99% of the normal gene and 1% TGT mutant gene; 99% of the normal gene and 1% GAT mutant gene; 99% of the normal gene and 1% GCT mutant gene; and 99% of the normal gene and 1% GTT mutant gene.

Gene amplification was conducted by repeating the PCR procedure of Example 1 by using 1 pg/µl of the template solutions and the fragment solution comprising 100% normal gene (GGT) for the template, and labeled NH₂-N-12S (100 ng) and NH₂-N-12A (100 ng) for the primers to prepare 7 types in total of the non-labeled sample DNAs.

### Competitive hybridization

Competitive hybridization was conducted by using the PCR solution comprising 0.125 µl of the labeled standard DNA mixed with 20 µl (excessiveness, 160) of the non-labeled sample DNA. Other components of the reaction solution and the reaction conditions were as in the case of the Example 1. In this case, competitive hybridization for contrast purpose was conducted by using water instead of the non-labeled sample DNA.

25 µl of the reaction solution was aliquoted, and the amount of the rehybridized labeled standard DNA was evaluated by means of ED-PCR as the intensity of the color development. The results are shown in Tables 6 and 7. The results are shown in Table 6 as the percentage (index value) when the intensity of the developed color in the case of the water was 100. The results are shown in Table 7 as the percentage (index value) when the intensity of the developed color in the case of the specimen ("normal") containing 100% of normal gene (GGT) was 100.

**Table 6**

| Labeled standard DNA | Non-labeled sample DNA | | | | | | | |
|---|---|---|---|---|---|---|---|---|
| | H₂O | normal | 1% AGT | 1% CGT | 1% TGT | 1% GAT | 1% GCT | 1% GTT |
| GGT | 100.0 | 2.3 | 2.5 | 1.8 | 1.7 | 2.2 | 1.5 | 2.6 |
| AGT | 100.0 | 53.2 | 19.3 | 50.9 | 54.4 | 49.4 | 52.7 | 50.2 |
| CGT | 100.0 | 76.2 | 70.7 | 26.9 | 71.4 | 72.1 | 77.4 | 71.4 |
| TGT | 100.0 | 45.1 | 44.9 | 41.9 | 14.1 | 43.2 | 42.3 | 44.9 |
| GAT | 100.0 | 44.7 | 44.8 | 43.1 | 42.6 | 18.1 | 43.2 | 46.4 |
| GCT | 100.0 | 53.8 | 53.4 | 50.5 | 52.7 | 53.0 | 18.0 | 51.6 |
| GTT | 100.0 | 42.0 | 38.9 | 41.2 | 41.6 | 39.4 | 40.1 | 18.0 |

**Table 7**

| Labeled standard DNA | Non-labeled sample DNA | | | | | | | |
|---|---|---|---|---|---|---|---|---|
| | H₂O | normal | 1% AGT | 1% CGT | 1% TGT | 1% GAT | 1% GCT | 1% GTT |
| GGT | - | - | - | - | - | - | - | - |
| AGT | - | 100 | 36 | 96 | 102 | 93 | 99 | 94 |
| CGT | - | 100 | 93 | 35 | 94 | 95 | 102 | 94 |
| TGT | - | 100 | 100 | 93 | 31 | 96 | 94 | 100 |
| GAT | - | 100 | 100 | 96 | 95 | 41 | 97 | 104 |
| GCT | - | 100 | 99 | 94 | 98 | 99 | 34 | 96 |
| GTT | - | 100 | 93 | 98 | 99 | 94 | 95 | 43 |

As shown in the results of Table 6, the specimen containing 1% of AGT mutant gene exhibited reduced color development intensity and reduced smaller index values for the normal gene (GGT) and for the AGT labeled standard DNA while no decrease in the index value was observed for other labeled standard DNAs. Similarly, the specimens containing the mutant gene in a minute amount were found to exhibit smaller index values for the normal gene (GGT) and for the labeled standard DNAs of the corresponding type.

As demonstrated above, the assay procedure of the present invention has been confirmed to be capable of detecting and identifying the mutant gene of a minute amount in the specimen at the content of as low as about several %.

As shown in Table 7, the index values of the specimens for the corresponding labeled standard DNA were in the range of 31 to 43, and it was then indicated that the content of the mutant gene is about 1% on the bases of the theoretical index values shown in Table 1 and the calibration curve of FIG. 2 to confirm the consistency with the actual content. Therefore, the procedure as described above is capable of quantatively determining the content of the mutant or polymorphic gene.

### [Example 5]

Labeled substituted standard DNAs of all substitution patterns wherein the 61st codon of the human chromosomal N-ras gene is substituted at one base such that the substitution results in the amino acid substitution were prepared, and the procedure of Example 1 was repeated to thereby detect and identify the gene mutation as described below.

Substitutions of the 61st codon CAA (Gln) of the normal N-ras gene at one base which result in the substitution of the amino acid include the following 7 substitutions: AAA (Lys), GAA (Glu), CCA (Pro), CGA (Arg), CTA (Leu), CAC (His) and CAT (His).

### Primers

The primers used for the gene amplification had the base sequence as described below.
N-61S
   SEQ ID No: 10
   5'-GTTGGACATACTGGATACAGCT-3'
N-61A
   SEQ ID No: 11
   5'-GTCTCTCATGGCACTGTACTCT-3'

### Preparation of labeled standard DNA

1 pg/µl solutions of the fragments respectively corresponding to the wild (CAA) gene and the mutant genes (8 types in total) were prepared by using ras Mutant set, N-ras codon 61 manufactured by Takara. Gene amplification by PCR was conducted by repeating the procedure of Example 1 using 1 pg of the fragment for the template, and Bio-N-61A (100 ng) and DNP-N-61S (100 ng) prepared by labeling the above-described primers for the primers to prepare 8 types in total of the labeled standard DNAs.

### Preparation of non-labeled sample DNA

The non-labeled DNAs were prepared as described below. The fragments containing the mutant gene were respectively mixed with the fragment having the base sequence of the normal gene (CAA) to prepare 7 types of templates each containing a minute amount of the mutant gene: the templates comprising 99% of the normal gene and 1% AAA mutant gene; 99% of the normal gene and 1% GAA mutant gene; 99% of the normal gene and 1% CCA mutant gene; 99% of the normal gene and 1% CGA mutant gene; 99% of the normal gene and 1% CTA mutant gene; 99% of the normal gene and 1% CAC mutant gene; and 99% of the normal gene and 1% CAT mutant gene.

Gene amplification was conducted by repeating the PCR procedure of Example 1 by using 1 pg/µl of the template solutions and the fragment solution comprising 100% normal gene for the template, and labeled NH₂-N-61S (100 ng) and NH₂-N-61A (100 ng) for the primers to prepare 8 types in total of the non-labeled sample DNAs.

### Competitive hybridization

Competitive hybridization was conducted by using the PCR solution comprising 0.125 µl of the labeled standard DNA mixed with 20 µl (excessiveness, 160) of the non-labeled sample DNA. Other components of the reaction solution and the reaction conditions were as in the case of the Example 1. In this case, the temperature gradient was such that the reaction solution was cooled from 89°C to 78°C in 80 minutes. The competitive hybridization for contrast purpose was conducted by using water instead of the non-labeled sample DNA.

25 µl of the reaction solution was aliquoted, and the amount of the rehybridized labeled standard DNA was evaluated by means of ED-PCR as the intensity of the color development. The results are shown in Tables 8 and 9. The results are shown in Table 8 as the percentage (index value) when the intensity of the developed color in the case of the water was 100. The results are shown in Table 9 as the percentage (index value) when the intensity of the developed color in the case of the specimen ("normal") containing 100% of normal gene (CAA) was 100.

**Table 8**

| Labeled standard DNA | Non-labeled sample DNA | | | | | | | | |
|---|---|---|---|---|---|---|---|---|---|
| | H₂O | normal | 1% AAA | 1% GAA | 1% CCA | 1% CGA | 1% CTA | 1% CAC | 1% CAT |
| CAA | 100.0 | 4.7 | 2.3 | 1.0 | 1.6 | 2.7 | 0.3 | 1.4 | 1.9 |
| AAA | 100.0 | 55.0 | 19.7 | 55.1 | 54.9 | 51.1 | 53.3 | 54.7 | 52.5 |
| GAA | 100.0 | 61.3 | 60.9 | 24.5 | 57.4 | 57.8 | 60.5 | 58.1 | 60.4 |
| CCA | 100.0 | 51.1 | 48.3 | 49.1 | 17.1 | 52.4 | 49.1 | 50.1 | 52.5 |
| CGA | 100.0 | 45.6 | 44.4 | 42.9 | 42.7 | 18.7 | 43.7 | 43.1 | 47.2 |
| CTA | 100.0 | 38.3 | 37.6 | 37.0 | 37.5 | 35.8 | 14.2 | 37.3 | 36.5 |
| CAC | 100.0 | 57.6 | 58.3 | 56.0 | 54.9 | 57.3 | 60.0 | 23.6 | 55.7 |
| CAT | 100.0 | 42.4 | 41.3 | 40.3 | 41.8 | 40.6 | 41.7 | 41.6 | 16.1 |

**Table 9**

| Labeled standard DNA | Non-labeled sample DNA | | | | | | | | |
|---|---|---|---|---|---|---|---|---|---|
| | H₂O | normal | 1% AAA | 1% GAA | 1% CCA | 1% CGA | 1% CTA | 1% CAC | 1% CAT |
| CAA | - | - | - | - | - | - | - | - | - |
| AAA | - | 100 | 36 | 100 | 100 | 93 | 97 | 100 | 95 |
| GAA | - | 100 | 99 | 40 | 94 | 94 | 99 | 95 | 99 |
| CCA | - | 100 | 95 | 96 | 33 | 103 | 96 | 98 | 103 |
| CGA | - | 100 | 97 | 94 | 94 | 41 | 96 | 95 | 103 |
| CTA | - | 100 | 98 | 97 | 98 | 94 | 37 | 98 | 95 |
| CAC | - | 100 | 101 | 97 | 95 | 99 | 104 | 41 | 97 |
| CAT | - | 100 | 98 | 95 | 99 | 96 | 98 | 98 | 38 |

As shown in the results of Table 8, the specimen containing 1% of AAA mutant gene exhibited reduced color development intensity and reduced smaller index values for the normal gene (CAA) and for the AAA labeled standard DNA while no decrease in the index value was observed for other labeled standard DNAs. Similarly, the specimens containing the mutant gene in a minute amount were found to exhibit smaller index values for the normal gene (CAA) and for the labeled standard DNAs of the corresponding type.

As demonstrated above, the assay procedure of the present invention has been confirmed to be capable of detecting and identifying the mutant gene of a minute amount in the specimen at the content of as low as about several %.

As shown in Table 9, the index values of the specimens for the corresponding labeled standard DNA were in the range of 33 to 41, and it was then indicated that the content of the mutant gene is about 1% on the bases of the theoretical index values shown in Table 1 and the calibration curve of FIG. 2 to confirm the consistency with the actual content. Therefore, the procedure as described above is capable of quantatively determine the content of the mutant or polymorphic gene.

As described above, the assay process of the present invention is capable of reliably detecting the mutant or polymorphic gene even when such mutant or polymorphic gene includes a substitution of only one base or when such mutant or polymorphic gene is present only in a minute amount in a large amount of normal gene. The assay process of the present invention has also enabled identification, and furthermore, quantification of the mutant or polymorphic gene which has so far been difficult.

The assay kit of the present invention is capable of rapidly detecting the mutant or polymorphic gene of a minute amount in the specimen in accordance with the assay process of the present invention by a convenient, reliable operation.

As described above, a reliable detection of mutation and polymorphism in a minute amount of gene in the specimen has been enabled, and the identification and quantification of the gene exhibiting mutation or polymorphism are also realized by the present invention. The present invention will enable tremendous advancement in the discovery, diagnosis and treatment of cancer and various virus and bacterial infections in their early stage, improvement in the success rate of bone marrow transplantation, qualitative and quantitative determination of the rejection, and the like.

## Claims

1. A nucleic acid assay process comprising the steps of amplifying a particular region of an analyte nucleic acid in a specimen to prepare a double stranded sample DNA; adding an excessive amount of said sample DNA to a labeled standard DNA comprising a double stranded nucleic acid having a site capable of binding to a solid support on one strand and a detectable label on the other strand to allow competitive hybridization to take place; and detecting the rehybridized labeled standard DNA by utilizing said detectable label and said site capable of binding to a solid support to thereby evaluate the degree of exchange of the complementary strands between said sample DNA and said labeled standard DNA for detecting the target DNA which is the same as said labeled standard DNA and which is present in said sample DNA;
characterized in that
a detection limit for the target DNA which is the same as said labeled standard DNA and which is present in said sample DNA is preliminarily selected, and excessiveness of said sample DNA added to said labeled standard DNA in the competitive hybridization is selected in accordance with the thus selected detection limit.

2. A nucleic acid assay process according to claim 1 wherein, when the detection limit for the target DNA which is the same as said labeled standard DNA and which is present in said sample DNA is A/B, the excessiveness of said sample DNA is at least B/A.

3. A nucleic acid assay process according to claim 1 or 2 wherein the target DNA which is the same as said labeled standard DNA and which is present in said sample DNA is quantitated by utilizing theoretical values of the degree of exchange of the complementary strands between said sample DNA and said labeled standard DNA at the selected excessiveness of said sample DNA.

4. A nucleic acid assay process according to any one of claims 1 to 3 wherein the analyte nucleic acid is a cancer-related gene, a gene related to genetic disease, a virus gene, a bacteria gene, or a polymorphic host gene.

5. A nucleic acid assay process according to any one of claims 1 to 4 wherein the analyte nucleic acid is k-ras gene, N-ras gene, p53 gene, BRCA1 gene, BRCA2 gene, or APC gene which is a cancer-related gene.

6. A nucleic acid assay process according to any one of claims 1 to 5 wherein the sample DNA is the one which has been gene amplified by using a pair of primers.

7. A nucleic acid assay process according to any one of claims 1 to 6 wherein the labeled standard DNA is the one prepared by gene amplification using a primer having introduced therein a detectable label and a primer having introduced therein a region capable of binding to a solid support.

8. A nucleic acid assay process according to any one of claims 1 to 6 wherein the labeled standard DNA is the one prepared by chemical synthesis.

9. A nucleic acid assay kit for assaying a nucleic acid in accordance with the nucleic acid assay process of any one of claims 1 to 8, characterized in that said kit includes
a labeled standard DNA comprising a double stranded nucleic acid having a site capable of binding to a solid support on one strand and a detectable label on the other strand.

10. A nucleic acid assay kit according to claim 9 comprising
a pair of primers for amplifying a particular region of an analyte nucleic acid in a specimen;
reagents for amplifying the particular region of the analyte nucleic acid in the specimen by using said primers to prepare the sample DNA;
a support for trapping the hybridizate; and
a reagent for detecting the hybridizate.
